# EUROPÄISCHE PATENTSCHRIFT

(11) **EP 1 924 670 B1**
(45) Veröffentlichungstag und Bekanntmachung des Hinweises auf die Patenterteilung: **02.08.2017**
(21) Anmeldenummer: 06776861.4
(22) Anmeldetag: 16.08.2006
(51) Int. Cl.: C09K 11/06, H05B 33/14, C07D 471/16, C09B 17/00, C09B 19/00, C09B 21/00, C09B 23/14, H01L 51/50, H01L 51/00, C09B 57/00

(54) **VERBINDUNGEN FÜR ORGANISCHE ELEKTRONISCHE VORRICHTUNGEN**
COMPOUNDS FOR ORGANIC ELECTRONIC DEVICES
COMPOSES POUR DISPOSITIFS ELECTRONIQUES ORGANIQUES

(30) Priorität: 12.09.2005 DE 102005043163
(43) Veröffentlichungstag der Anmeldung: 28.05.2008
(60) Teilanmeldung: 10008854.1
(73) Patentinhaber: Merck Patent GmbH, 64293 Darmstadt (DE)
(72) Erfinder: PARHAM, Amir, 65929 Frankfurt (DE); HEUN, Susanne, 65812 Bad Soden (DE); VESTWEBER, Horst, 34630 Gilersberg-Winterscheid (DE); STOESSEL, Philipp, 60487 Frankfurt (DE); HEIL, Holger, 64295 Darmstadt (DE); FORTTE, Rocco, 65933 Frankfurt (DE)
(86) Internationale Anmeldenummer: PCT/EP2006/008053
(87) Internationale Veröffentlichungsnummer: WO 2007/031165

(56) Entgegenhaltungen:
- WO-A-99/20081
- WO-A-2004/070772
- WO-A1-2006/080640
- DE-A1- 19 808 088
- JP-A- 1 076 061
- JP-A- 5 107 784
- JP-A- S6 476 061
- JP-A- 11 339 868
- JP-A- H05 107 784
- JP-A- H11 339 868
- HELLWINKEL D ET AL: "ZWEIFACH ORTHO-VERBRUECKTE TRIPHENYLAMIN-DERIVATE DOUBLY ORTHO-BRIDGED TRIPHENYLAMINE DERIVATIVES" CHEMISCHE BERICHTE, VERLAG CHEMIE GMBH. WEINHEIM, DE, Bd. 113, Nr. 1, 1980, Seiten 358-384, XP009063905 ISSN: 0009-2940 in der Anmeldung erwähnt
- FIELD J E; HILL T J; VENKATARAMAN D: "Bridged Triarylamines: A New Class of Heterohelicenes" JOURNAL OF ORGANIC CHEMISTRY, Bd. 68, 2003, Seiten 6071-6078, XP002425435 in der Anmeldung erwähnt
- KURATSU M; KOZAKI M; OKADA K: "2,2':6',2'':6'',6-Trioxytriphenylamine: Synthesis and Properties of the Radical Cation and Neutral Species" ANGEWANDTE CHEMIE, Bd. 117, 31. Mai 2005 (2005-05-31), Seiten 4124-4126, XP002425436 in der Anmeldung erwähnt
- KOENE B E ET AL: "Unsymmetrical triaryldiamines as thermally stable hole transporting layers for organic light-emitting devices" CHEMISTRY OF MATERIALS, AMERICAN CHEMICAL SOCIETY, WASHINGTON, US, Bd. 10, Nr. 8, 1998, Seiten 2235-2250, XP002979930 ISSN: 0897-4756
- FIELD J E ET AL: "Bridged Triarylamines: A New Class of Heterohelicenes", THE JOURNAL OF ORGANIC CHEMISTRY, AMERICAN CHEMICAL SOCIETY, US, vol. 68, 1 January 2003 (2003-01-01), pages 6071-6078, XP002425435, ISSN: 0022-3263, DOI: 10.1021/JO026883P
- KOENE B E ET AL: "Unsymmetrical triaryldiamines as thermally stable hole transporting layers for organic light-emitting devices", CHEMISTRY OF MATERIALS, AMERICAN CHEMICAL SOCIETY, US, vol. 10, no. 8, 1 January 1998 (1998-01-01), pages 2235-2250, XP002979930, ISSN: 0897-4756, DOI: 10.1021/CM980186P
- KURATSU M ET AL: "2,2':6',2'':6'',6-Trioxytriphenylamine: Synthesis and Properties of the Radical Cation and Neutral Species", ANGEWANDTE CHEMIE, WILEY - V C H VERLAG GMBH & CO. KGAA, DE, vol. 117, 1 January 2005 (2005-01-01), pages 4124-4126, XP002425436, ISSN: 0044-8249, DOI: 10.1002/ANGE.200500397
- HELLWINKEL D ET AL: "ZWEIFACH ORTHO-VERBRUECKTE TRIPHENYLAMIN-DERIVATE//DOUBLY ORTHO-BRIDGED TRIPHENYLAMINE DERIVATIVES", CHEMISCHE BERICHTE, VCH, DE, vol. 113, no. 1, 1 January 1980 (1980-01-01), pages 358-384, XP009063905, ISSN: 0009-2940

## Beschreibung

Die vorliegende Erfindung beschreibt neue Verbindungen und deren Einsatz in organischen Elektrolumineszenzvorrichtungen.

Organische Halbleiter werden für eine Reihe verschiedenartiger Anwendungen, die im weitesten Sinne der Elektronikindustrie zugerechnet werden können, entwickelt. Der Aufbau organischer Elektrolumineszenzvorrichtungen (OLEDs), in denen diese organischen Halbleiter als funktionelle Materialien eingesetzt werden, ist beispielsweise in US 4539507, US 5151629, EP 0676461 und WO 98/27136 beschrieben. Allerdings zeigen diese Vorrichtungen noch erhebliche Probleme, die einer dringenden Verbesserung bedürfen:
1. Die Effizienz ist gerade bei fluoreszierenden OLEDs immer noch zu niedrig und muss verbessert werden.
2. Die operative Lebensdauer ist insbesondere bei blauer Emission noch nicht ausreichend, so dass bis dato nur einfache Anwendungen kommerziell realisiert werden konnten.
3. Die Betriebsspannung ist gerade bei fluoreszierenden OLEDs recht hoch und sollte daher weiter verringert werden, um die Leistungseffizienz zu verbessern. Das ist insbesondere für mobile Anwendungen von großer Bedeutung.
4. Viele blau emittierende Emitter, die aromatische Amine enthalten, sind thermisch nicht stabil und zersetzen sich beim Sublimieren oder beim Aufdampfen. Dadurch ist die Verwendung dieser Systeme nicht bzw. nur unter großen Verlusten und mit hohem technischen Aufwand möglich.

Materialien basierend auf Arylaminen werden wegen ihrer physikalischen, photochemischen und elektrochemischen Eigenschaften eingehend als Lochtransport- bzw. Emittermaterialien untersucht; sie bilden einheitliche amorphe Schichten und können stabile Radikalkationen bilden, ohne sich chemisch zu verändern.

Organische Verbindungen, die sich als Lochleiter bewährt haben, leiten sich im Allgemeinen vom Diarylamino-substituierten Triphenylamin (TPA-Typ), vom Diarylamino-substituierten Biphenyl (TPD-Typ) oder Kombinationen dieser Grundverbindungen (TPTE-Typen) ab. Weiterhin erweisen sich auch Tristilbenamine (z. B. TSA und MSA) mit Phenylenvinylen-Strukturelementen als geeignete Materialien, die eine effiziente Lochleitung gewährleisten.

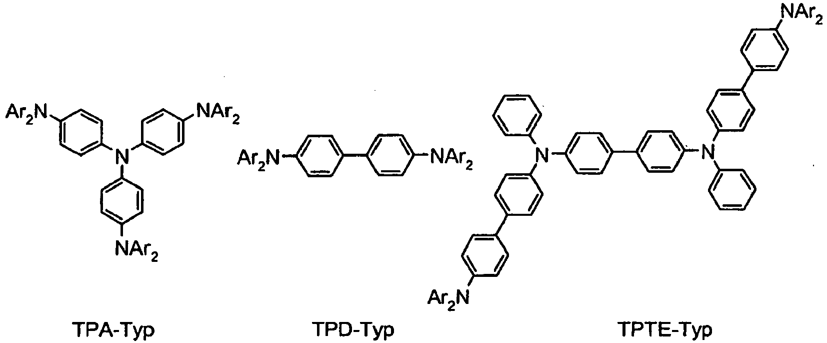

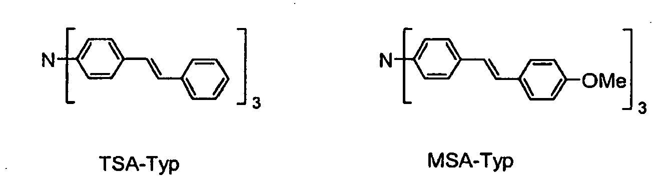

Überraschend wurde nun gefunden, dass eine neue Klasse von Triarylaminen nochmals verbesserte elektronische Eigenschaften aufweist. Diese Verbindungen enthalten eine starre planare Triphenylamineinheit und flexible Strukturelemente in der äußeren Peripherie, wodurch die Flexibilität des Molekülzentrums verringert und die Löslichkeit durch Substituenten erhöht wird; außerdem können durch Erweiterung des π-Elektronensystems in der äußeren Peripherie Verbindungen synthetisiert werden, die als Emitter eingesetzt werden können. Darüber hinaus können planare stabile Triarylamin-Radikalkationen Anwendung als elektronische und magnetische Materialien finden.

Eine Reihe zweifach und dreifach *ortho*-verbrückter Triphenylamine wurde bereits in den siebziger Jahren synthetisiert, aber nicht eingehend auf ihre physikalischen Eigenschaften untersucht (D. Hellwinkel, M. Melan, Chem. Ber. 1974, 107, 616-626; D. Hellwinkel, W. Schmidt, Chem. Ber. 1980, 113, 358-384). Eine weitere Entwicklung hierzu ist die Synthese des unsubstituierten Triphenylamin-Grundgerüsts, das in *ortho*-Position über Keto-Gruppen verbrückt ist (J. E. Field, T. J. Hill, D. Venkataraman, J. Org. Chem. 2003, 68, 6071-6078; J. E. Field, D. Venkataraman, Chem. Mater. 2002, 14, 962-964), sowie die Synthese des unsubstituierten Triphenylamin-Grundgerüsts, das in *ortho*-Position über Ether-Gruppen verbrückt ist. Diese Verbindungen bilden stabile Radikalkationen (M. Kuratsu, M. Kozaki, K. Okada, Angew. Chem. 2005, 117, 4124-4126). Ein Nachteil dieser Verbindungen, insbesondere derjenige, die über Keto-Gruppen verknüpft sind, ist jedoch ihre sehr geringe Löslichkeit in gängigen organischen Lösemitteln, was eine effiziente Reinigung durch Umkristallisation oder Chromatographie erschwert oder verhindert. Dies gilt insbesondere für die Reinigung größerer Mengen, wie sie in der Displayfertigung benötigt werden.

Überraschend wurde gefunden, dass die erfindungsgemäßen Verbindungen ausgezeichnete Eigenschaften als Emitter und Lochleiter in OLEDs aufweisen.

Die erfindungsgemäßen Verbindungen absorbieren und emittieren langwelliger als die analogen nicht-verbrückten Vertreter. Bei der Verbrückung der Phenylamin-Einheit wird eine deutlich intensivere Emission beobachtet. Weiterhin weisen diese Verbindungen eine höhere Lochmobilität auf.

Die erfindungsgemäßen Verbindungen sind reproduzierbar in hoher Reinheit herstellbar und weisen keine Chargenschwankung auf. Ein industrieller Prozess zur Herstellung der erfindungsgemäßen Elektrolumineszenzvorrichtungen ist daher wesentlich effizienter.

Die erfindungsgemäßen Verbindungen zeichnen sich durch ihre gute Löslichkeit in organischen Lösungsmitteln aus, was ihre Reinigung und Verarbeitung erheblich erleichtert. Damit sind diese Verbindungen auch aus Lösung durch Beschichtungs- oder Drucktechniken verarbeitbar. Auch bei der üblichen Verarbeitung durch Verdampfen ist diese Eigenschaft von Vorteil, da so die Reinigung der Anlagen bzw. der eingesetzten Schattenmasken erheblich erleichtert wird. Die erfindungsgemäßen Verbindungen zeichnen sich weiterhin durch eine verbesserte Oxidationsstabilität in Lösung aus, was sich zum einen positiv auf die Reinigung und zum anderen generell auf die Handhabung dieser Verbindungen auswirkt, da die Lagerstabilität von Lösungen, die zur Verarbeitung mit Druckverfahren hergestellt wurden, deutlich verbessert ist. Die erfindungsgemäßen Verbindungen zeichnen sich außerdem durch eine hohe Temperaturstabilität aus, so dass sie im Hochvakuum unzersetzt verdampft werden können. Diese Eigenschaft ist eine Grundvoraussetzung zur reproduzierbaren Darstellung von OLED-Devices und wirkt sich insbesondere positiv auf die operative Lebensdauer aus.

Diese Verbindungen und deren Verwendung in OLEDs sind daher der Gegenstand der vorliegenden Erfindung.

Gegenstand der Erfindung sind Verbindungen der Formel (2) sowie eine organische Elektrolumineszenzvorrichtung, enthaltend Verbindungen gemäß Formel (1) und Formel (2), 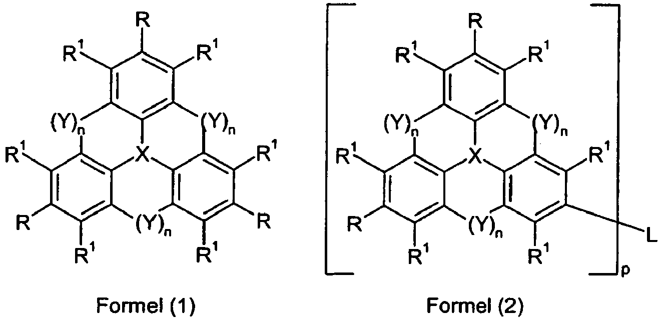 wie in den Patentansprüchen 1 und 10 beschrieben.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (bevorzugt weniger als 10 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, etc. als aromatische Ringsysteme im Sinne dieser Erfindung verstanden werden.

Unter einer Arylgruppe bzw. einer Heteroarylgruppe im Sinne dieser Erfindung wird eine aromatische Gruppe bzw. heteroaromatische Gruppe mit einem gemeinsamen aromatischen Elektronensystem verstanden. Dies kann im Sinne dieser Erfindung ein einfacher Homo- oder Heterocyclus sein, beispielsweise Benzol, Pyridin, Thiophen, etc., oder es kann ein kondensiertes aromatisches Ringsystem sein, in dem mindestens zwei aromatische oder heteroaromatische Ringe, beispielsweise Benzolringe, miteinander "verschmolzen", d. h. durch Anellierung einander ankondensiert sind, also mindestens eine gemeinsame Kante und dadurch auch ein gemeinsames aromatisches System aufweisen. Diese Aryl- oder Heteroarylgruppen können substituiert oder unsubstituiert sein; ebenso können gegebenenfalls vorhandene Substituenten weitere Ringsysteme bilden. So sind beispielsweise Systeme wie Naphthalin, Anthracen, Phenanthren, Pyren, etc. als Arylgruppen und Chinolin, Acridin, Benzothiophen, Carbazol, etc. als Heteroarylgruppen im Sinne dieser Erfindung zu sehen, während beispielsweise Biphenyl, Fluoren, Spirobifluoren, etc. keine Arylgruppen darstellen, da es sich hierbei um separate aromatische Elektronensysteme handelt.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Trifluormethoxy Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 40 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren; Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Beispiele für bevorzugte Verbindungen gemäß Formel (1) sind die im Folgenden abgebildeten Strukturen (1) bis (48).

| | |
|---|---|
| 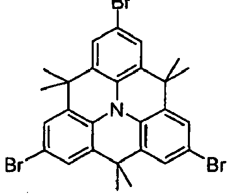 | 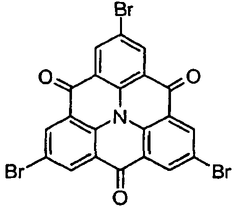 |
| Struktur (1) | Struktur (2) |
| 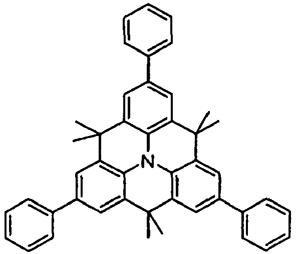 | 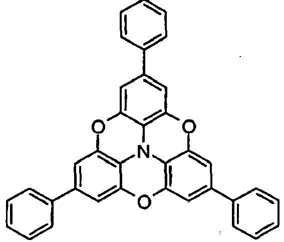 |
| Struktur (3) | Struktur (4) |
| 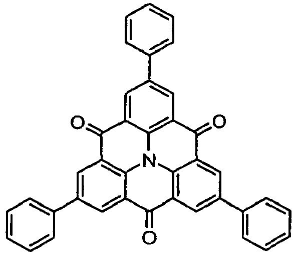 | 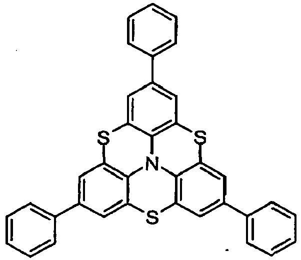 |
| Struktur (5) | Struktur (6) |
| 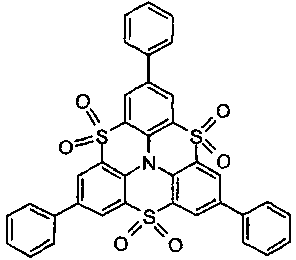 | 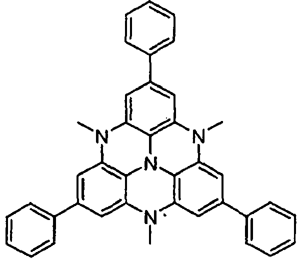 |
| Struktur (7) | Struktur (8) |
| 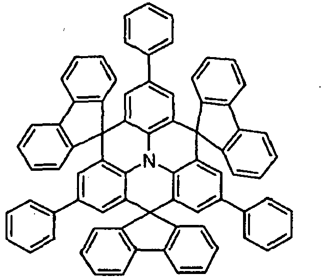 | 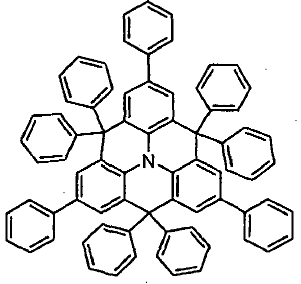 |
| Struktur (9) | Struktur (10) |
| 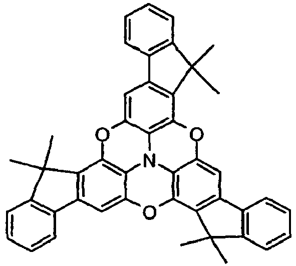 | 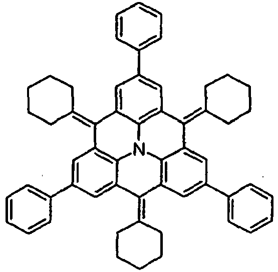 |
| Struktur (11) | Struktur (12) |
| 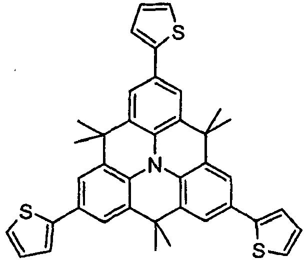 | 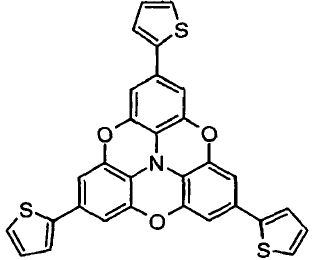 |
| Struktur (13) | Struktur (14) |
| 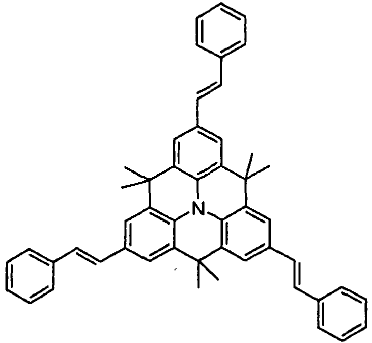 | 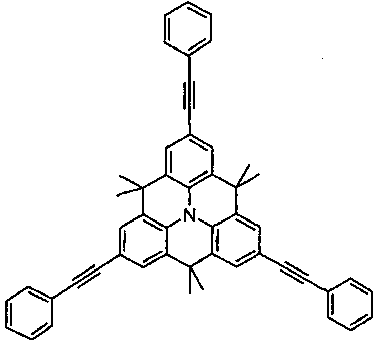 |
| Struktur (15) | Struktur (16) |
| 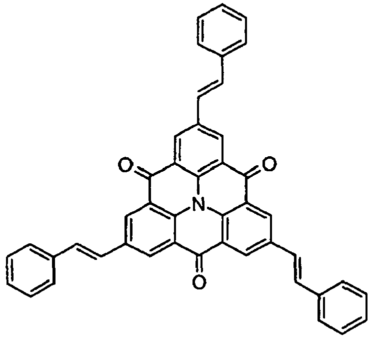 | 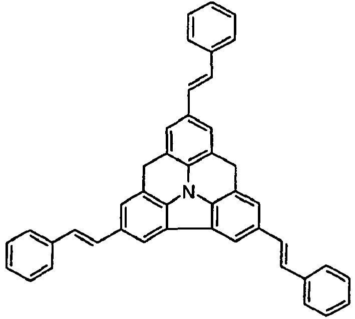 |
| Struktur (17) | Struktur (18) |
| 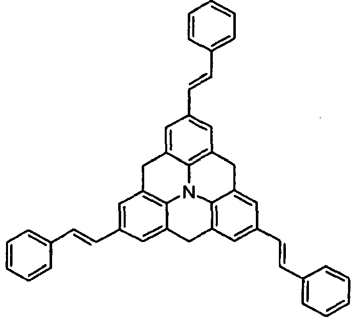 | 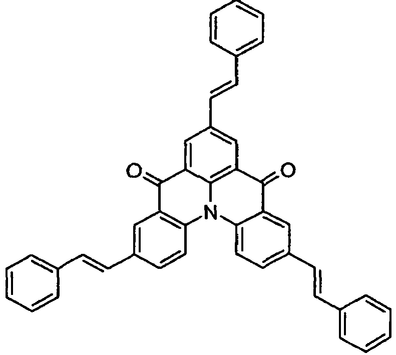 |
| Struktur (19) | Struktur (20) |
| 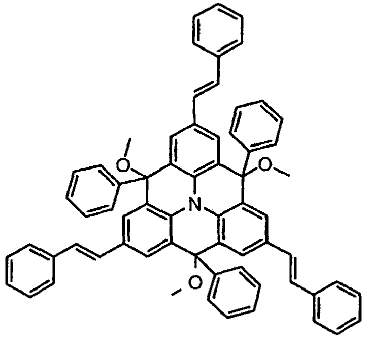 | 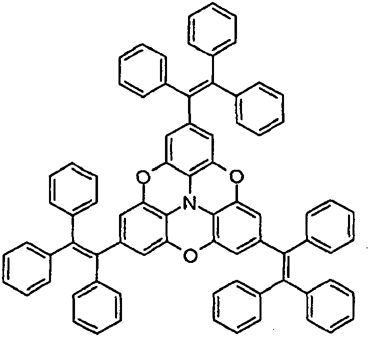 |
| Struktur (21) | Struktur (22) |
| 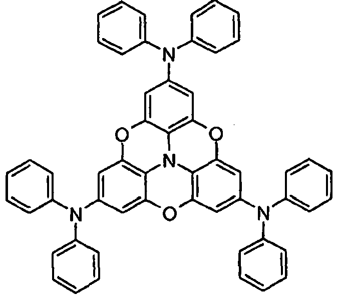 | 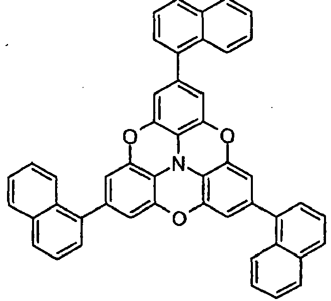 |
| Struktur (23) | Struktur (24) |
| 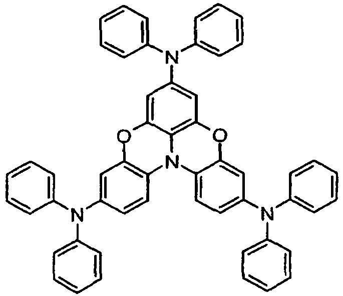 | 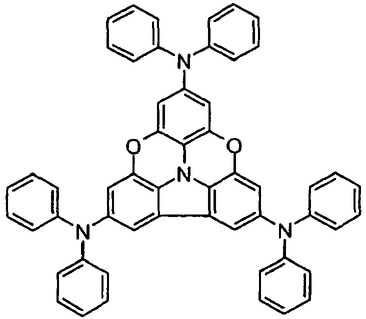 |
| Struktur (25) | Struktur (26) |
| 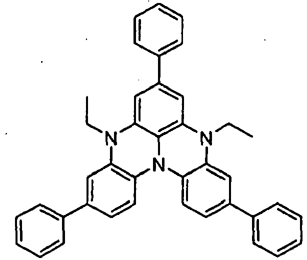 | 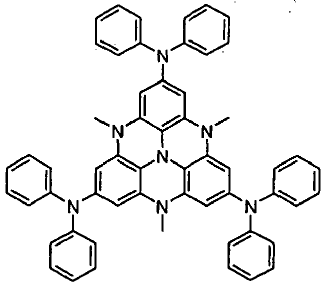 |
| Struktur (27) | Struktur (28) |
| 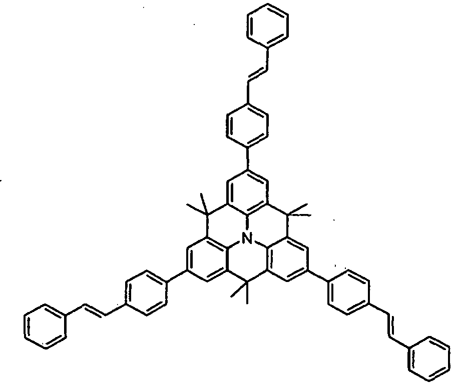 | |
| Struktur (29) | |
| 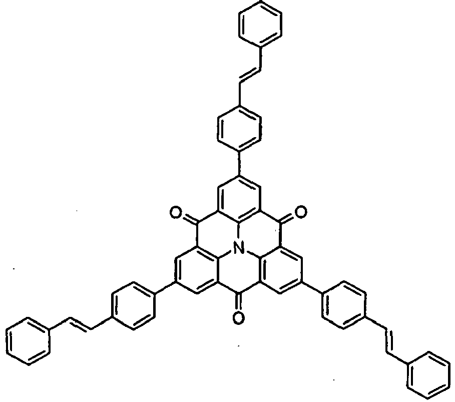 | |
| Struktur (30) | |
| 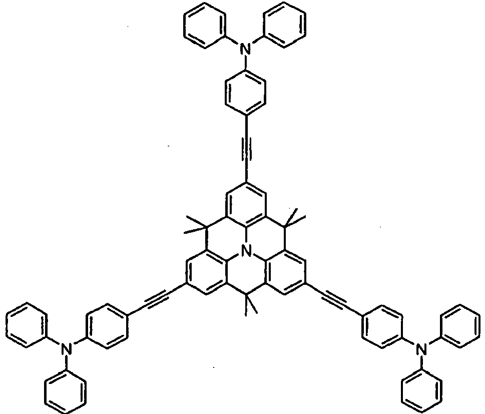 | |
| Struktur (31) | |
| 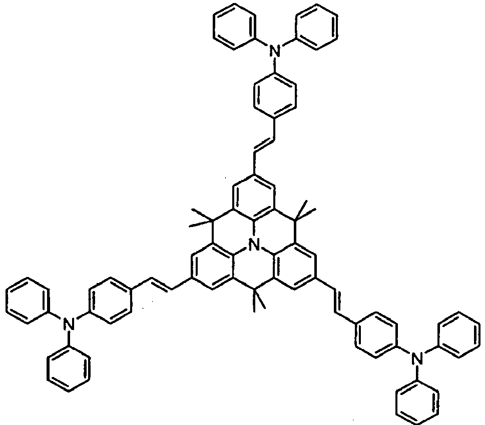 | |
| Struktur (32) | |
| 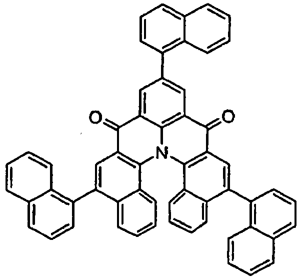 | |
| Struktur (33) | |
| 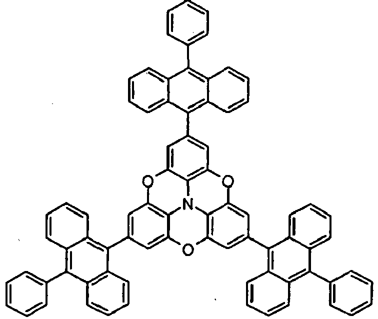 | |
| Struktur (34) | |
| 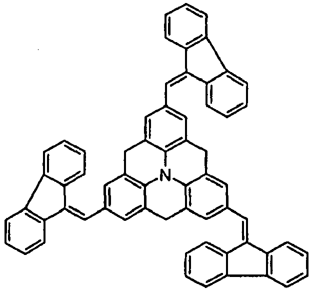 | 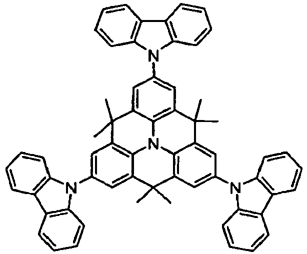 |
| Struktur (35) | Struktur (36) |
| 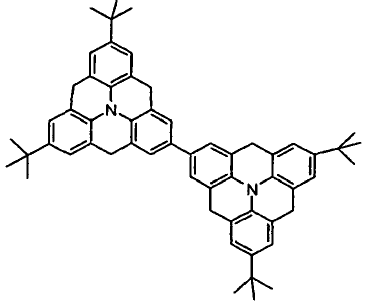 | |
| Struktur (37) | |
| 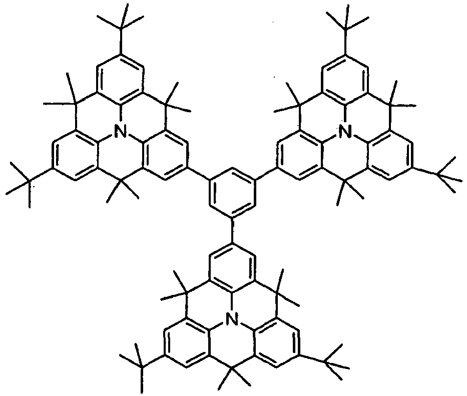 | |
| Struktur (38) | |
| 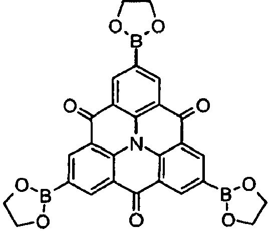 | 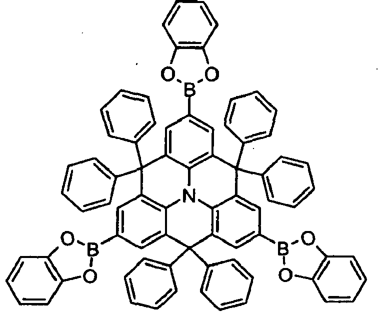 |
| Struktur (39) | Struktur (40) |
| 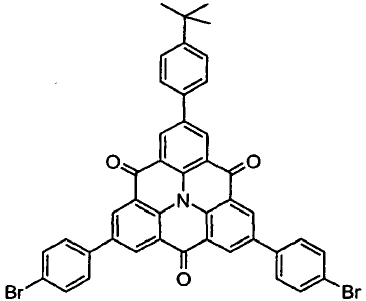 | |
| Struktur (41) | |
| 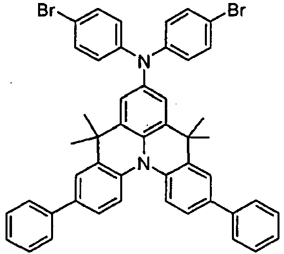 | |
| Struktur (42) | |
| 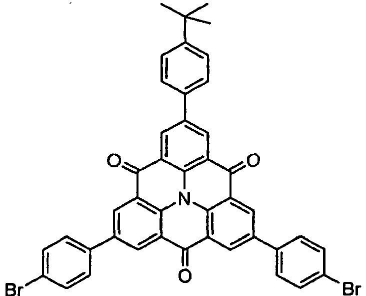 | |
| Struktur (43) | |
| 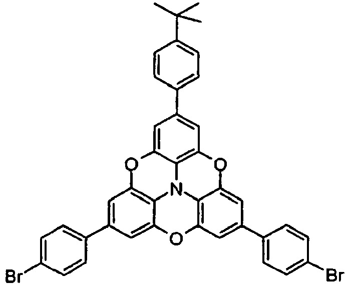 | |
| Struktur (44) | |
| 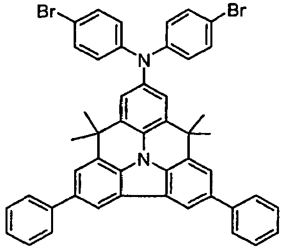 | |
| Struktur (45) | |
| 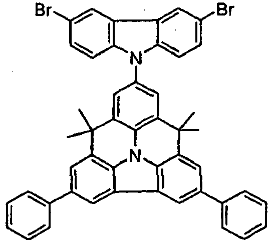 | |
| Struktur (46) | |
| 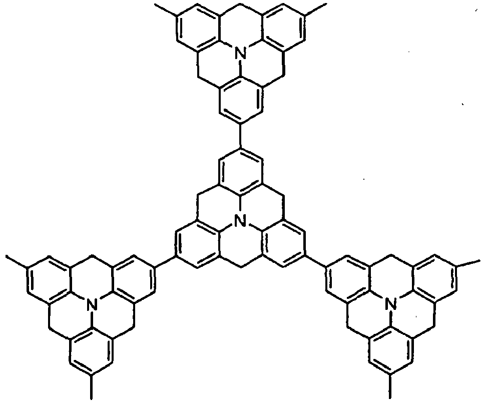 | |
| Struktur (47) | |
| 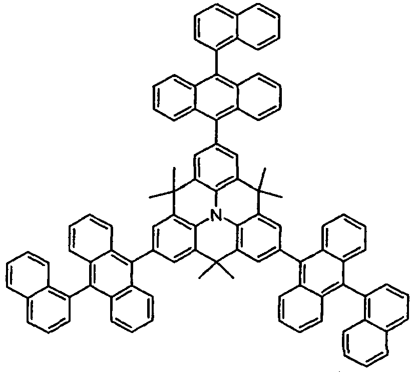 | |
| Struktur (48) | |

Durch gezielte Wahl der verbrückenden Einheit Y können die physikalischen Eigenschaften beeinflusst und optimiert werden. Das bestimmende Strukturelement dieser neuen Verbindungsklasse ist die planare Triphenylamineinheit mit seinem niedrigen Ionisationspotential, während die Substituenten und die Wahl der Brücke Y für die Emission und für die festkörperphysikalischen Parameter (Schmelzpunkt, Glaspunkt, amorphes Verhalten) entscheidend sind.

Durch die Derivatisierung und Erweiterung des π-Systems im planaren Triphenylaminsystem kann die Wechselwirkung der Bausteine, je nach Wahl der Substituenten, beeinflusst werden und somit eine Steigerung der Löslichkeit ermöglicht werden.

Die Fähigkeit zur Bildung säulenförmiger π-Stapel durch die Planarität der zentralen Einheiten macht diese Molekülklasse weiterhin auch zu einem idealen konjugierten, elektroaktiven Baustein für dreidimensionale Netzwerke und diskotische Flüssigkristalle.

Bevorzugte Ausführungen der erfindungsgemäßen Verbindungen sind solche, bei denen die Glasübergangstemperatur T_{g} größer als 90 °C, bevorzugt größer als 100 °C, besonders bevorzugt größer als 120 °C ist.

Die Grundgerüste der erfindungsgemäßen Verbindungen können nach dem Fachmann bekannten Syntheseschritten, wie z. B. Bromierung, Ullmann-Arylierung, Hartwig-Buchwald-Kupplung, etc., dargestellt werden, wie in Schema 1 und Schema 2 dargestellt.

Diese Grundgerüste können in einem weiteren Schritt funktionalisiert werden. So führt die Bromierung von Triphenylaminen mit Brückenelementen zu Tri-p-brom-substituierten verbrückten Triphenylaminen, wobei hier, bedingt durch den +M-dirigierenden Effekt des Stickstoffatoms, gute Ausbeuten bei exzellenten Regioselektivitäten erreicht werden. Als Bromierungsagens können neben elementarem Brom vor allen auch N-Brom-Verbindungen wie N-Brom-succinimid (NBS) verwendet werden. Ebenso geeignet ist die Funktionalisierung mit anderen reaktiven Gruppen, beispielsweise Chlor, Iod, Boronsäure bzw. Boronsäurederivate, Triflat oder Tosylat.

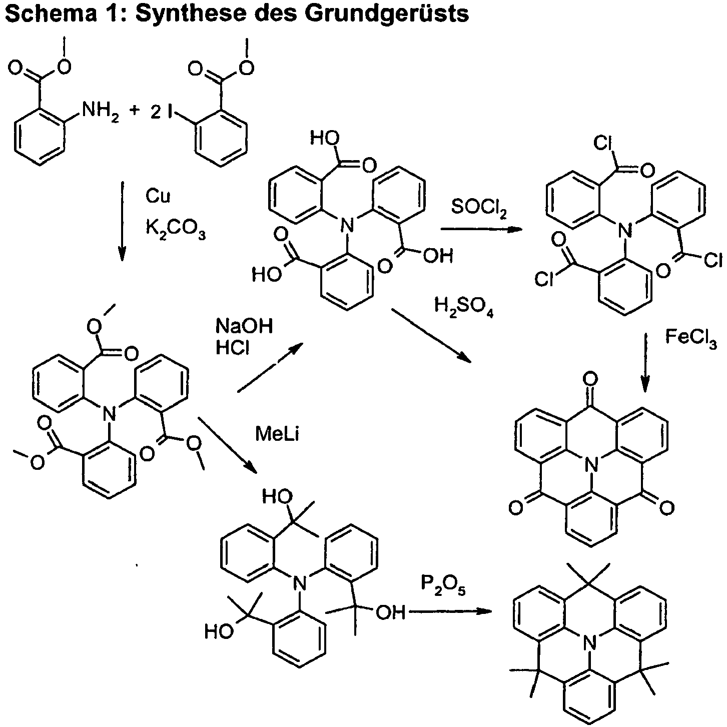

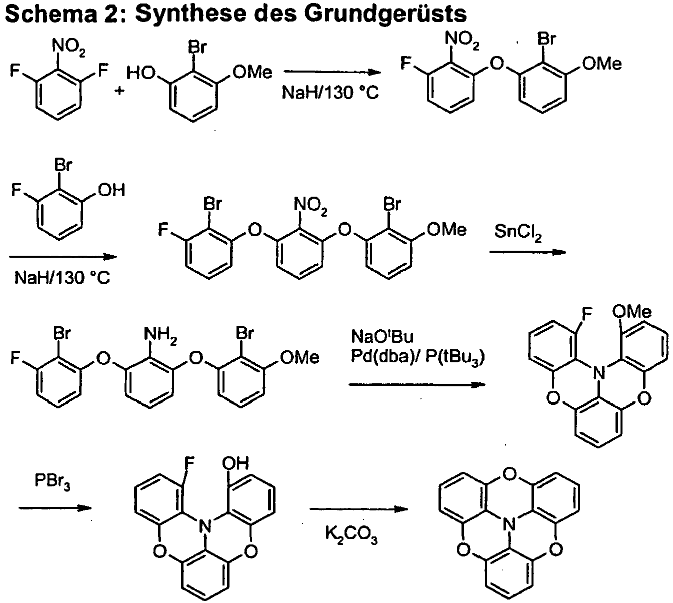

Die funktionalisierten, insbesondere bromierten Verbindungen stellen den zentralen Baustein für die weitere Funktionalisierung dar, wie in Schema 3 dargestellt. So lassen sich diese funktionalisierten verbrückten Verbindungen beispielsweise durch Suzuki-Kupplung mit funktionalisierten Arylboronsäuren in Verbindungen gemäß Formel (1) umsetzen. Ebenso können andere Kupplungsreaktionen (z. B. Stille-Kupplung, Heck-Kupplung, Sonogashira-Kupplung, etc.) Verwendung finden. Kupplung mit Diarylaminen nach Hartwig-Buchwald führt zu Triarylamin-Derivaten. Entsprechend können aliphatische Amine, Carbazole, etc. als Substituenten eingeführt werden. Als Funktionalisierung kommen weiterhin Formyl-, Alkylcarbonyl- und Arylcarbonyl-Gruppen oder deren geschützte Analoga, z. B. in Form der entsprechenden Dioxolane, in Frage. Die so erhaltenen Carbonyl-Substrate können leicht, z. B. durch eine Wittig-Horner-Reaktion, in die entsprechenden Olefine überführt werden. Die bromierten Verbindungen können weiterhin lithiiert und durch Reaktion mit Elektrophilen wie Benzonitril und anschließender saurer Hydrolyse zu Ketonen oder mit Chlordiphenylphosphinen und anschließender Oxidation zu Phosphinoxiden umgesetzt werden.

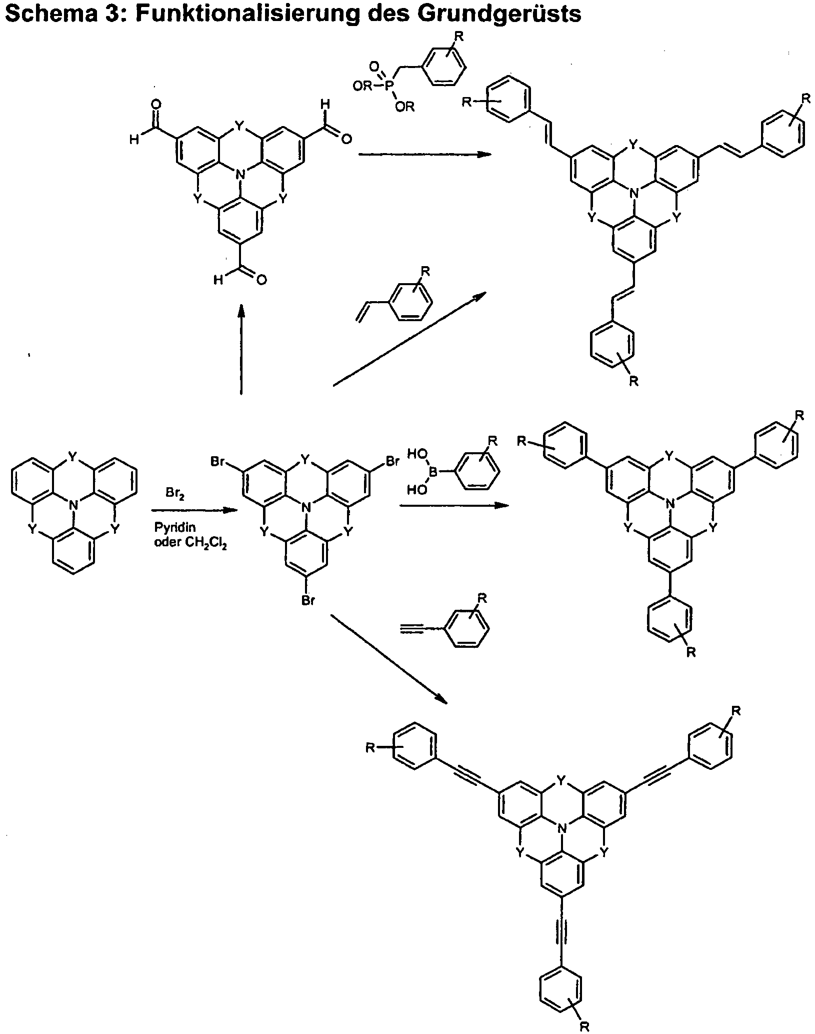

Weiterer Gegenstand der Erfindung ist daher ein Verfahren zur Herstellung von Verbindungen gemäß Formel (1), dadurch gekennzeichnet, dass der nicht funktionalisierte Grundkörper gemäß Formel (1), in dem alle Reste R für Wasserstoff stehen, funktionalisiert, insbesondere bromiert wird, und in einem weiteren Schritt die Substituenten R eingeführt werden.

Geeignet funktionalisierte Verbindungen gemäß Formel (1) oder Formel (2), insbesondere bromierte Verbindungen, wie beispielsweise die oben abgebildeten Strukturen (41) bis (46), können auch zum Einbau in Polymere verwendet werden.

Gegenstand der Erfindung sind daher weiterhin Polymere, Oligomere oder Dendrimere, enthaltend Wiederholeinheiten gemäß Formel (1) oder Formel (2). Diese Polymere, Oligomere oder Dendrimere können konjugiert, teilkonjugiert oder nicht-konjugiert sein. Unter einem Oligomer im Sinne dieser Anmeldung soll eine Verbindung mit mindestens drei Wiederholeinheiten verstanden werden.

Weitere Wiederholeinheiten der Polymere, Oligomere oder Dendrimere sind bevorzugt gewählt aus der Gruppe bestehend aus Fluorenen (z. B. gemäß EP 842208 oder WO 00/22026), Spirobifluorenen (z. B. gemäß EP 707020, EP 894107 oder EP 04028865.6), Para-phenylenen (z. B. gemäß WO 92/18552), Dihydrophenanthrenen (z. B. gemäß WO 05/014689), Phenanthrenen (z. B. gemäß WO 05/104264 oder der nicht offen gelegten Anmeldung DE 102005037334.3), Indenofluorenen (z. B. gemäß WO 04/041901 oder WO 04/113412), Carbazolen (z. B. gemäß WO 04/070772), Anthracenen, Naphthalinen oder Thiophenen (z. B. gemäß EP 1028136). Weitere bevorzugte Wiederholeinheiten sind fluoreszierende oder phosphoreszierende emittierende Einheiten, beispielsweise basierend auf Vinylarylaminen oder Metallkomplexen, oder lochleitende Einheiten, insbesondere basierend auf Triarylaminen. Auch Polymere mit mehreren dieser Einheiten oder Homopolymere der Wiederholeinheiten gemäß Formel (1) sind möglich.

Die Verbindungen gemäß Formel (1) und Formel (2) können in organischen elektronischen Vorrichtungen, insbesondere in organischen Elektrolumineszenzvorrichtungen, eingesetzt werden. Unter einer organischen elektronischen Vorrichtung im Sinne dieser Erfindung wird eine Vorrichtung verstanden, welche Anode, Kathode und mindestens eine Schicht aufweist, welche mindestens eine organische Verbindung enthält. In organischen Elektrolumineszenzvorrichtungen ist mindestens eine Schicht eine emittierende Schicht. Außer Kathode, Anode und der emittierenden Schicht kann die organische Elektrolumineszenzvorrichtung noch weitere Schichten enthalten. Diese können beispielsweise sein: Lochinjektionsschicht, Lochtransportschicht, Elektronentransportschicht und/oder Elektroneninjektionsschicht. Es sei aber an dieser Stelle darauf hingewiesen, dass nicht notwendigerweise jede dieser Schichten vorhanden sein muss.

Je nach Substitution wird die Verbindung gemäß Formel (1) bzw. Formel (2) in unterschiedlichen Funktionen bzw. in unterschiedlichen organischen Schichten eingesetzt.

In einer bevorzugten Ausführungsform der Erfindung wird die Verbindung in der emittierenden Schicht als Mischung mit mindestens einem Hostmaterial eingesetzt. Dies ist insbesondere dann der Fall, wenn mindestens ein Substituent R der Verbindung der Formel (1) bzw. Formel (2) eine Gruppe CR²=CR²Ar darstellt, bevorzugt mindestens zwei Substituenten R, besonders bevorzugt alle drei Substituenten R. Weiterhin eignen sich hier besonders Verbindungen gemäß Formel (2), in denen das Symbol L für eine Gruppe -CR²=CR²- steht.

Unter einem Hostmaterial wird in einem System aus Host und Dotand diejenige Komponente verstanden, die in dem System im höheren Anteil vorliegt. Bei einem System aus einem Host und mehreren Dotanden wird als Host diejenige Komponente verstanden, deren Anteil der höchste in der Mischung ist.

Der Anteil des Hostmaterials in der emittierenden Schicht beträgt zwischen 50.0 und 99.9 Gew.-%, bevorzugt zwischen 80.0 und 99.5 Gew.-%, besonders bevorzugt zwischen 90.0 und 99.0 Gew.-%. Entsprechend beträgt der Anteil des Dotanden gemäß der Formel (1) bzw. Formel (2) zwischen 0.1 und 50.0 Gew.-%, bevorzugt zwischen 0.5 und 20.0 Gew.-%, besonders bevorzugt zwischen 1.0 und 10.0 Gew.-%.

Als Hostmaterialien kommen verschiedene Stoffklassen in Frage. Bevorzugte Hostmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911), der elektronenleitenden Verbindungen, insbesondere Ketone, Phosphinoxide, Sulfoxide, etc. (z. B. gemäß WO 05/084081 und WO 05/084082) oder der Atropisomere (z. B. gemäß der nicht offen gelegten Anmeldung EP 04026402.0). Besonders bevorzugte Hostmaterialien sind ausgewählt aus den Klassen der Oligoarylene, enthaltend Naphthalin, Anthracen und/oder Pyren oder Atropisomeren dieser Verbindungen, der Oligoarylenvinylene, der Ketone, der Phosphinoxide und der Sulfoxide. Ganz besonders bevorzugte Hostmaterialien sind ausgewählt aus den Klassen der Oligoarylene, enthaltend Anthracen und/oder Pyren oder Atropisomeren dieser Verbindungen, der Phosphinoxide und der Sulfoxide. Unter einem Oligoarylen im Sinne dieser Anmeldung soll eine Verbindung verstanden werden, in der mindestens drei Aryl- bzw. Arylengruppen aneinander gebunden sind.

Weiterhin bevorzugt sind organische Elektrolumineszenzvorrichtungen, dadurch gekennzeichnet, dass mehrere emittierende Verbindungen in derselben Schicht oder in unterschiedlichen Schichten verwendet werden, wobei mindestens eine dieser Verbindungen eine Struktur gemäß Formel (1) bzw. Formel (2) aufweist. Besonders bevorzugt weisen diese Verbindungen insgesamt mehrere Emissionsmaxima zwischen 380 nm und 750 nm auf, so dass insgesamt weiße Emission resultiert, d. h. außer der Verbindung gemäß Formel (1) bzw. Formel (2) wird noch mindestens eine weitere emittierende Verbindung verwendet, die fluoreszieren oder phosphoreszieren kann und die gelbes, orange oder rotes Licht emittiert. Insbesondere bevorzugt sind Dreischichtsysteme, wovon mindestens eine dieser Schichten eine Verbindung gemäß Formel (1) bzw. Formel (2) enthält und wobei die Schichten blaue, grüne und orange oder rote Emission zeigen (für den prinzipiellen Aufbau siehe z. B. WO 05/011013).

In einer weiteren bevorzugten Ausführungsform der Erfindung wird die Verbindung gemäß Formel (1) bzw. Formel (2) als Hostmaterial in einer emittierenden Schicht eingesetzt. Dies ist insbesondere dann der Fall, wenn die Substituenten R für aromatische oder heteroaromatische Ringsysteme stehen.

In nochmals einer weiteren bevorzugten Ausführungsform der Erfindung wird die Verbindung gemäß der Formel (1) bzw. der Formel (2) als Lochtransportmaterial bzw. als Lochinjektionsmaterial in einer organischen Elektrolumineszenzvorrichtung oder einer anderen organischen elektronischen Vorrichtung eingesetzt. Die Verbindungen sind dann bevorzugt mit aromatischen Substituenten R oder Gruppen der Formel N(Ar)₂ substituiert. Die Verbindung wird bevorzugt in einer Lochtransport- bzw. in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen einer Lochinjektionsschicht und einer Emissionsschicht liegt. Wenn die Verbindungen gemäß Formel (1) oder Formel (2) als Lochtransport- bzw. als Lochinjektionsmaterial verwendet werden, kann es bevorzugt sein, wenn sie mit Elektronenakzeptor-Verbindungen dotiert werden, beispielsweise mit F₄-TCNQ oder mit Verbindungen, wie in EP 1476881 oder EP 1596445 beschrieben. Verbindungen gemäß Formel (1) und Formel (2) weisen sehr hohe Lochbeweglichkeiten auf, die möglicherweise durch Stapeleffekte der Verbindungen erzeugt werden. Somit kann diese Verbindungsklasse auch zur Erzeugung anisotroper Transporteigenschaften herangezogen werden.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig. Hohe Löslichkeit lässt sich durch geeignete Substitution der Verbindungen erreichen.

Die erfindungsgemäßen Verbindungen weisen bei Verwendung in organischen Elektrolumineszenzvorrichtungen folgende überraschende Vorteile gegenüber dem Stand der Technik auf:
1. Die Effizienz entsprechender Vorrichtungen wird höher im Vergleich zu Systemen gemäß dem Stand der Technik, insbesondere im Vergleich zu Systemen, die keine Brückenelemente enthalten.
2. Die Stabilität entsprechender Vorrichtungen wird höher im Vergleich zu Systemen gemäß dem Stand der Technik, was sich vor allem in einer deutlich höheren Lebensdauer zeigt.
3. Die Verbindungen lassen sich gut und ohne erhebliche Zersetzung sublimieren und aufdampfen, sind dadurch leichter zu verarbeiten und deshalb besser für die Verwendung in OLEDs geeignet als Materialien gemäß dem Stand der Technik.
4. Der Stokes-Shift (Differenz zwischen Absorption und Emission) ist kleiner als bei verwandten Verbindungen in unverbrückter Struktur.
5. Die Verbindungen weisen eine hohe Ladungsträgerbeweglichkeit auf.

Im vorliegenden Anmeldetext und auch in den im Weiteren folgenden Beispielen wird auf die Verwendung der erfindungsgemäßen Verbindungen in Bezug auf OLEDs und PLEDs und die entsprechenden Displays abgezielt. Trotz dieser Beschränkung der Beschreibung ist es für den Fachmann ohne weiteres erfinderisches Zutun möglich, die erfindungsgemäßen Verbindungen auch für weitere Verwendungen in anderen elektronischen Vorrichtungen zu benutzen, z. B. für organische Feld-Effekt-Transistoren (O-FETs), organische Dünnfilmtransistoren (O-TFTs), organische lichtemittierende Transistoren (O-LETs), organische integrierte Schaltungen (O-ICs), organische Solarzellen (O-SCs), organische Feld-Quench-Devices (O-FQDs), lichtemittierende elektrochemische Zellen (LECs), organische Photorezeptoren oder organische Laserdioden (O-Laser). Die Verwendung der erfindungsgemäßen Verbindungen in den entsprechenden Vorrichtungen ebenso wie diese Vorrichtungen selbst sind ebenfalls ein Gegenstand der vorliegenden Erfindung.

Die Erfindung durch die nachfolgenden Beispiele näher erläutert wird, ohne sie dadurch einschränken zu wollen.

### Beispiele:

Die nachfolgenden Synthesen werden, sofern nicht anders angegeben, unter einer Schutzgasatmosphäre durchgeführt. Die Edukte können von den Firmen ALDRICH bzw. ABCR (Anthranilsäuremethylester, 2-lodbenzoesäuremethylester Palladium(II)acetat, Tri-o-tolylphosphin, Anorganika, Lösemittel) bezogen werden. Die Synthese von 4,4,8,8,12,12-Hexamethyl-2,6,10-tribrom-4H,8H,12H-benzo[1,9]chinolizino[3,4,5,6,7-defg]acridin ist in der Literatur (D. Hellwinkel, M. Melan, Chem. Ber. 1974, 107, 616-626) beschrieben.

### Beispiel 1: Synthese von 4,4,8,8,12,12-Hexamethyl-2,6,10-tribrom-4H,8H,12H-benzo[1,9]chinolizino[3,4,5,6,7,-defg]acridin

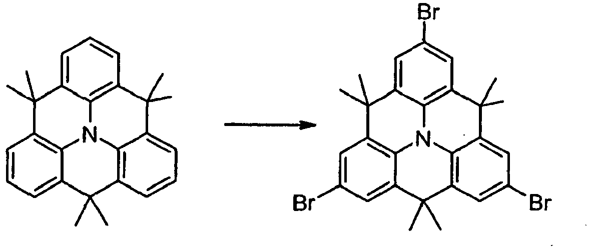

5.3 g (14.5 mmol) 4,4,8,8,12,12- Hexamethyl-4H,8H,12H-benzo[1,9]-chinolizino-[3,4,5,6,7,-defg]acridin werden in 150 mL CH₂Cl₂ vorgelegt. Anschließend tropft man unter Lichtausschluss bei 0 °C eine Lösung aus 8.0 g (45.1 mmol) NBS in 100 ml CH₂Cl₂ hinzu, lässt auf RT kommen und rührt 4 h weiter bei dieser Temperatur. Anschließend wird die Mischung mit 150 mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt. Ausbeute: 3 g (5 mmol), 57 % d. Th., Reinheit nach ¹H-NMR ca. 98 %. Diese Verbindung wird als Intermediat für die Folgesynthesen eingesetzt.

### Beispiel 2: Synthese von 4,4,8,8,12,12-Hexamethyl-2,6,10-triphenyl-4H,8H,12H-benzo[1,9]chinolizino[3,4,5,6,7,-defg]acridin (HTM1)

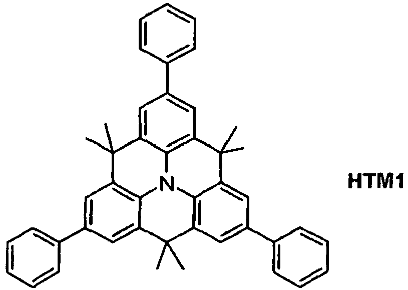

Ein entgaste Suspension von 3 g (5 mmol) 2,6,10-Tribrom- 4,4,8,8,12,12-Hexamethyl-4H,8H,12H-benzo[1,9]chinolizino[3,4,5,6,7,-*defg*]acridin (aus Beispiel 1), 2.74 g (22.5 mmol) Phenylboronsäure und 7.8 g (31.5 mmol) Kaliumphosphat-hydrat in einem Gemisch aus 7.5 ml Dioxan, 15 ml Toluol und 18 ml Wasser wird unter gutem Rühren mit 0.27 g (0.9 mmol) Tri-o-tolylphosphin und dann mit 33.5 mg (0.15 mmol) Palladium(II)acetat versetzt. Nach 5 h Kochen unter Rückfluss lässt man die Mischung erkalten. Der Niederschlag wird abgesaugt, dreimal mit 10 ml Ethanol / Wasser (1:1, v:v) und dreimal mit 5 ml Ethanol gewaschen und anschließend im Vakuum getrocknet. Ausbeute: 2.4 g (4 mmol), 81 % d. Th., Reinheit nach ¹H-NMR ca. 98 %.

### Beispiel 3: Synthese des Dotanden D1

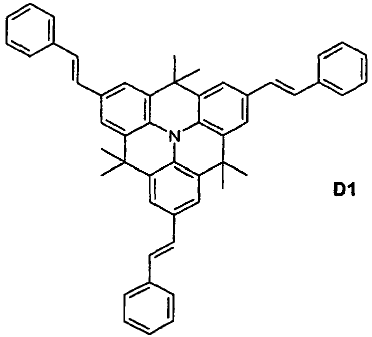

Ein Gemisch aus 30.1 g (50 mmol) 4,4,8,8,12,12-Hexamethyl-2,6,10-tribrom-4H,8H,12H-benzo[1,9]chinolizino[3,4,5,6,7,-*defg*]acridin (aus Beispiel 1), 25.8 ml (225 mmol) Styrol, 337 mg (1.5 mmol) Palladium(II)-acetat, 1.55 g (15 mmol) N,N-Dimethylglycin, 81 mg (0.5 mmol) Eisen(III)-chlorid und 37.8 g (450 mmol) Natriumhydrogencarbonat in 500 ml NMP wird langsam unter gutem Rühren auf 140 °C erhitzt und anschließend 16 h bei dieser Temperatur gerührt. Nach Erkalten werden 500 ml Dichlormethan und 1000 ml Wasser zugesetzt. Die organische Phase wird abgetrennt und fünfmal mit je 500 ml Wasser gewaschen. Nach Trocknen über Natriumsulfat wird die organische Phase zur Trockene eingeengt. Der so erhaltene gelbe Feststoff wird sechsmal aus Toluol umkristallisiert, dann zweimal mit je 500 ml Ethanol unter Rückfluss ausgerührt. Es werden 22 g (32 mmol) (entsprechend 68 % d. Th.) des Produkts mit einer Reinheit > 99.4 % nach HPLC erhalten.

### Beispiel 4: Synthese der Hostmaterials H1

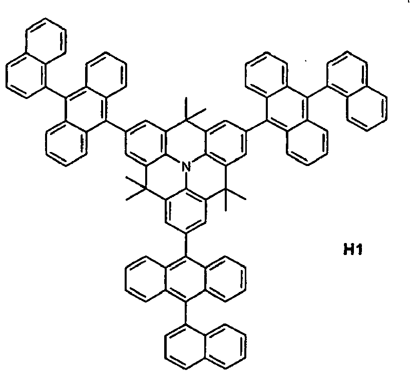

Zur einer mit Stickstoff gesättigten Mischung aus 6.2 g (10.4 mmol) 4,4,8,8,12,12-Hexamethyl-2,6,10-tribrom-4H,8H,12H-benzo[1,9]-chinolizino[3,4,5,6,7-defg]acridin (aus Beispiel 1), 9.9 g (31.2 mmol) 10-(1-naphthyl)-9-anthryl-Boronsäure, 14.9 g (70.2 mmol) K₃PO₄, 200 ml Dioxan und 200 ml Wasser werden 1.3 g (1.17 mmol) Pd(PPh₃)₄ gegeben, und die Suspension wird 7 h auf 80 °C erhitzt. Danach werden 0.08 g NaCN zugegeben, und die wässrige Phase wird abgetrennt. Die organische Phase wird zweimal mit H₂O gewaschen und über Na₂SO₄ getrocknet. Der so erhaltene Rückstand wird aus Toluol umkristallisiert. Die ausgefallenen Kristalle werden abgesaugt, mit 50 ml Ethanol gewaschen und im Vakuum getrocknet. Es werden 10.4 g (8.1 mmol) (entsprechend 80 % d. Th.) des Produkts mit einer Reinheit > 99.6 % nach HPLC erhalten.

### Beispiel 5: DFT (Dichte-Funktional-Theorie) Rechnungen

Näheren Aufschluss über die Natur planarer Triaraylamine geben die HOMO- und LUMO-Energien und die Bandlücken. Diese Größen lassen sich mit Elektronenaffinitäten, Ionisierungspotentialen, elektronischen Übergängen und Reaktivitäten korrelieren. Zur Berechnung der Energien dieser Moleküle im elektronischen Grundzustand wurden DFT (Dichte-Funktional-Theorie) Rechnungen durchgeführt (Tabelle 1). Dabei entsprechen die Strukturen den in der Beschreibung abgebildeten Strukturen. Die Anregungsenergien wurden über das HOMO-LUMO-Gap abgeschätzt. Es wurde mit dem Basissatz b3pw91 gerechnet. Außerdem sind die Rechnungen über Cyclovoltammetrie-Daten kalibriert.

**Tabelle 1: Ergebnisse der DFT-Rechnungen**

| **Beispiel** | **HOMO (Hartrees)** | **HOMO [eV]** | **HOMO Corrected** | **LUMO (HARTREES)** | **LUMO [eV]** | **LUMO Corrected** | **GAP [eV]** | **λmax-theo [nm]** |
|---|---|---|---|---|---|---|---|---|
| Struktur (3) | -0,17519 | -4,77 | -5,14 | -0,03394 | -0,92 | -2,11 | 3,02 | 411 |
| Struktur (19) | -0,17134 | -4,66 | -5,04 | -0,05484 | -1,49 | -2,52 | 2,52 | 493 |
| Struktur (23) | -0,16639 | -4,53 | -4,92 | -0,03124 | -0,85 | -2,06 | 2,86 | 434 |

### Beispiel 6: Herstellung der OLEDs

Die Herstellung der OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen werden die Ergebnisse verschiedener OLEDs vorgestellt. Der grundlegende Aufbau, die verwendeten Materialien und Schichtdicken, außer der emittierenden Schicht und der Lochinjektions- und Lochtransportschicht, sind zur besseren Vergleichbarkeit identisch. Analog dem o. g. allgemeinen Verfahren werden OLEDs mit folgendem Aufbau erzeugt:

| | |
|---|---|
| Lochinjektionsschicht (HIL) | 0 nm oder 20 nm **HTM1**, siehe Tabelle 1 |
| Lochtransportschicht (HTL) | 40 nm oder 20 nm NPB (aufgedampft; N-Naphthyl-N-phenyl-4,4'-diaminobiphenyl), siehe Tabelle 1 |
| Emissionschicht (EML) | siehe Tabelle 1 für Materialien, Konzentration 5 %, Schichtdicke 30 nm |
| Elektronenleiter (ETL) | 20 nm Alq₃ (bezogen von SynTec; Tris(chinolinato)aluminium(III)) |
| LiF-Al (Kathode) | 1 nm LiF, darauf 150 nm Al. |

Diese OLEDs werden standardmäßig charakterisiert; hierfür wurden die Elektrolumineszenzspektren, die Effizienz (gemessen in cd/A), die Leistungseffizienz (gemessen in lm/W) in Abhängigkeit der Helligkeit, berechnet aus Strom-Spannungs-Leuchtdichte-Kennlinien (IUL-Kennlinien), und die Lebensdauer bestimmt. Als Lebensdauer wird die Zeit definiert, nach der die Anfangshelligkeit von 1000 cd/m² auf die Hälfte gesunken ist.

In Tabelle 2 sind die Ergebnisse einiger OLEDs (Beispiele 7 bis 9) zusammengefasst, wobei jeweils die Zusammensetzung der EML, der HIL und der HTL inklusive der Schichtdicken mit aufgeführt ist. Beispiel 7 zeigt den Dotanden **D2** gemäß dem Stand der Technik in dem erfindungsgemäßen Hostmaterial **H1** (aus Beispiel 4). Beispiel 8 zeigt den Host **H2** gemäß dem Stand der Technik mit dem erfindungsgemäßen Emissionsmaterial **D1** (aus Beispiel 3). Die OLED in Beispiel 9 enthält zusätzlich **HTM1** (aus Beispiel 2) als Lochinjektionsmaterial. Die Strukturformeln des verwendeten Dotanden **D2** und des Hostmaterials H2 gemäß dem Stand der Technik sind im Folgenden dargestellt:

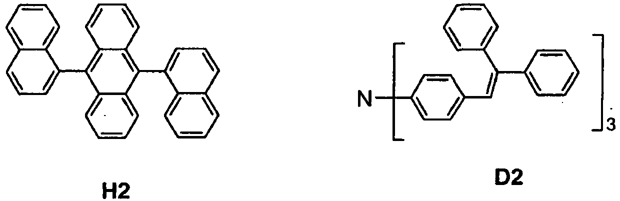

**Tabelle 2**

| Beispiel | HIL | HTL | EML | Max. Effizienz (cd/A) | Spannung (V) bei 1000cd/m² | CIE^{a} | Lebensdauer (h)^{b} |
|---|---|---|---|---|---|---|---|
| 7 | | **NPB** (40 nm) | **H1 : D2** (5%) (30 nm) | 8.0 | 6.0 | x=0.16 | 3500 |
| | | | | | | y=0.24 | |
| 8 | | **NPB** (40 nm) | **H2 : D1** (5%) (30 nm) | 4.1 | 6.5 | x=0.15 | 1500 |
| | | | | | | y=0.11 | |
| 9 | **HTM1** (20 nm) | **NPB** (20 nm) | **H2 : D1** (5%) (30 nm) | 4.1 | 6.5 | x=0.15 | 1700 |
| | | | | | | y=0.13 | |

| | | | | | | | |
|---|---|---|---|---|---|---|---|
| ^{a} CIE-Koordinaten: Farbkoordinaten der Commision Internationale de l'Eclairage 1931. ^{b} Lebensdauer: Zeit bis zum Abfall der Helligkeit auf 50 % der Anfangshelligkeit, gemessen bei einer Anfangshelligkeit von 1000 cd/m². | | | | | | | |

Zusammenfassend kann gesagt werden, dass sich die erfindungsgemäßen Verbindungen, beispielsweise die Verbindungen **HTM1, D1** und **H1,** sehr gut für die Verwendung in organischen Elektrolumineszenzvorrichtungen geeignet sind und dass organische Elektrolumineszenzvorrichtungen, welche diese Materialien enthalten, sehr gute Eigenschaften aufweisen.

## Patentansprüche

1. Organische Elektrolumineszenzvorrichtung, enthaltend Anode, Kathode und mindestens eine emittierende Schicht, **dadurch gekennzeichnet, dass** mindestens eine Schicht mindestens eine Verbindung gemäß Formel (1) oder Formel (2) enthält, 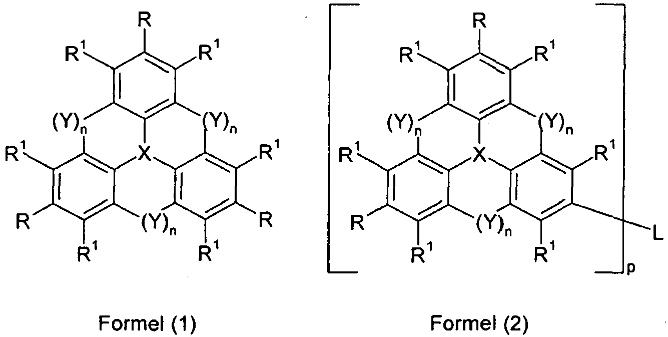 wobei für die verwendeten Symbole und Indizes gilt:
X ist bei jedem Auftreten gleich oder verschieden N, P, As, Sb, P=O, As=O oder Sb=O;
Y ist gleich oder verschieden bei jedem Auftreten O, S, C(R¹)₂, -C=C(R¹)₂, Si(R¹)₂, BR¹, NR¹, PR¹, AsR¹, SbR¹, BiR¹, P(=O)R¹, As(=O)R¹, Sb(=O)R¹, Bi(=O)R¹, SO, SeO, TeO, SO₂, SeO₂, TeO₂ oder eine chemische Bindung;
R¹ ist bei jedem Auftreten gleich oder verschieden H, OH, F, Cl, Br, I, CN, NO₂, N(Ar)₂, Si(R²)₃, B(OR²)₂, C(=O)Ar, P(=O)Ar₂, S(=O)Ar, S(=O)Ar₂, CR²=CR²Ar, C≡CAr, OSO₂R², eine geradkettige Alkyl gruppe mit 1 bis 40 C-Atomen oder eine verzweigte oder cyclische Akyl gruppe mit 3 bis 40 C-Atomen, die jeweils mit einem oder mehreren Resten R² substituiert sein kann, wobei eine oder mehrere nicht benachbarte CH₂-Gruppen durch -R²C=CR²-, -C≡C-, Si(R²)₂, Ge(R²)₂, Sn(R²)₂, C=O, C=S, C=Se, C=NR², P(=O)R², S=O, SO₂, NR², oder -CONR²- ersetzt sein können und wobei ein oder mehrere H-Atome durch F, Cl, Br, I, CN oder NO₂ ersetzt sein können, oder ein aromatisches oder heteroaromatisches Ringsystem mit 5 bis 40 aromatische Ringatomen, welches durch einen oder mehrere Reste R² substituiert sein kann, oder eine Kombination aus zwei, drei, vier oder fünf dieser Systeme; dabei können auch zwei oder mehrere Substituenten R¹ sowohl am selben Ring wie auch an unterschiedlichen Ringen miteinander ein mono- oder polycyclisches, aliphatisches, aromatisches oder heteroaromatisches Ringsystem bilden;
R ist definiert wie R¹, wobei mindestens ein Rest R ungleich Wasserstoff ist;
R² ist bei jedem Auftreten gleich oder verschieden H oder ein aliphatischer, aromatischer und/oder heteroaromatischer Kohlenwasserstoffrest mit 1 bis 20 C-Atomen, wobei auch zwei oder mehrere Reste R² miteinander ein Ringsystem bilden können;
Ar ist bei jedem Auftreten gleich oder verschieden ein aromatisches oder heteroaromatisches Ringsystem mit 5-30 aromatischen Ringatomen, das mit einem oder mehreren nicht-aromatischen Resten R¹ substituiert sein kann;
L ist eine mindestens bivalente geradkettige Alkylen-, Alkylenoxy- oder Thioalkylenoxygruppe mit 1 bis 40 C-Atomen oder eine verzweigte oder cyclische Alkylylen-, Alkylenoxy- oder Thioalkylenoxygruppe mit 3 bis 40 C-Atomen, die mit jeweils einem oder mehreren Resten R² substituiert sein kann, wobei eine oder mehrere nicht benachbarte CH₂-Gruppen durch -R²C=CR²-, -C≡C-, Si(R²)₂, Ge(R²)₂, Sn(R²)₂, C=O, C=S, C=Se, C=NR², P(=O)R², S=O, SO₂, -O-, -S- oder -CONR²- ersetzt sein können und wobei ein oder mehrere H-Atome durch F, Cl, Br, I, CN oder NO₂ ersetzt sein können, oder ein mindestens bivalentes aromatisches oder heteroaromatisches Ringsystem mit 5 bis 40 aromatischen Ringatomen, welches durch einen oder mehrere Reste R² substituiert sein kann, oder P(R¹)₃₋ₚ, P(=O)(R¹)₃₋ₚ, C(R¹)₄₋ₚ, Si(R¹)₄₋ₚ, N(Ar)₃₋ₚ oder eine Kombination aus zwei, drei, vier oder fünf dieser Systeme; oder L ist eine chemische Bindung;
n ist bei jedem Auftreten gleich oder verschieden 0, 1 oder 2, wobei n = 0 bedeutet, dass statt Y ein Wasserstoff oder Rest R¹ vorhanden ist, mit der Maßgabe, dass mindestens zwei Indizes n ungleich 0 sind;
p ist 2, 3, 4, 5 oder 6, mit der Maßgabe, dass p nicht größer ist als die maximale Valenz von L;
dabei ist die folgende Verbindung von der Erfindung ausgenommen:

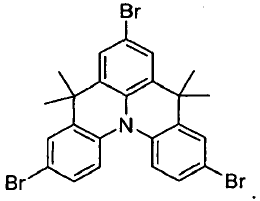

2. Organische Elektrolumineszenzvorrichtung gemäß Anspruch 1, **dadurch gekennzeichnet, dass** das Symbol X für Stickstoff, Phosphor oder P=O steht.

3. Organische Elektrolumineszenzvorrichtung gemäß Anspruch 1 oder 2, **dadurch gekennzeichnet, dass** die Symbole Y gleich oder verschieden bei jedem Auftreten für O, S, (CR¹)₂, P(=O)R¹, C=C(R¹)₂, NR¹, SO, SO₂ oder eine chemische Bindung stehen.

4. Organische Elektrolumineszenzvorrichtung gemäß einem oder mehreren der Ansprüche 1 bis 3, **dadurch gekennzeichnet, dass** das Symbol R¹ gleich oder verschieden bei jedem Auftreten für H, F, CF₃, einen aliphatischen, aromatischen oder heteroaromatischen Kohlenwasserstoffrest mit 1 bis 10 C-Atomen steht, oder dass R¹ an Brücken Y für einen aliphatischen Kohlenwasserstoffrest mit 1 bis 6 C-Atomen oder für eine Aryl- oder Heteroarylgruppe mit 6 bis 10 C-Atomen steht und zwei Reste R¹ an derselben Brücke Y miteinander ein Ringsystem bilden können.

5. Organische Elektrolumineszenzvorrichtung gemäß einem oder mehreren der Ansprüche 1 bis 4, **dadurch gekennzeichnet, dass** das Symbol R bei jedem Auftreten gleich oder verschieden für F, Cl, Br, I, CHO, B(OR²)₂, P(R²)₂, N(Ar)₂, R²C=CR²Ar, C≡CAr oder ein aromatisches oder heteroaromatisches Ringsystem mit 5 bis 30 aromatischen Ringatomen, das durch einen oder mehrere nicht-aromatische Reste R¹ substituiert sein kann, oder eine Kombination aus zwei, drei, vier oder fünf dieser Reste steht.

6. Organische Elektrolumineszenzvorrichtung gemäß einem oder mehreren der Ansprüche 1 bis 5, **dadurch gekennzeichnet, dass** das Symbol L für -R²C=CR²-, -C≡C-, C=O, S=O, SO₂, -O-, -S-, P(R¹)₃₋ₚ, P(=O)(R¹)₃₋ₚ, C(R¹)₄₋ₚ, Si(R¹)₄₋ₚ, N(Ar)₃₋ₚ oder ein aromatisches oder heteroaromatisches Ringsystem mit 6 bis 30 aromatischen Ringatomen, welches durch einen oder mehrere Reste R² substituiert sein kann, oder oder eine Kombination aus zwei, drei oder vier dieser Systeme oder eine chemische Bindung steht.

7. Organische Elektrolumineszenzvorrichtung enthaltend mindestens ein Polymer, Oligomer oder Dendrimer, enthaltend eine oder mehrere der Verbindungen gemäß Formel (1) oder Formel (2), wobei mindestens ein Rest R oder R¹ eine Bindung zum Polymer darstellt.

8. Organische Elektrolumineszenzvorrichtung gemäß einem oder mehreren der Ansprüche 1 bis 7, **dadurch gekennzeichnet, dass** die Verbindung gemäß Formel (1) oder Formel (2) als Mischung mit mindestens einem Hostmaterial eingesetzt wird und/oder dass die Verbindung gemäß Formel (1) oder Formel (2) als Lochtransport- und/oder Lochinjektionsmaterial eingesetzt wird.

9. Verwendung von Verbindungen gemäß Formel (1) oder Formel (2) gemäß Anspruch 1 in organischen Elektrolumineszenzvorrichtungen.

10. Verbindung gemäß Formel (2), 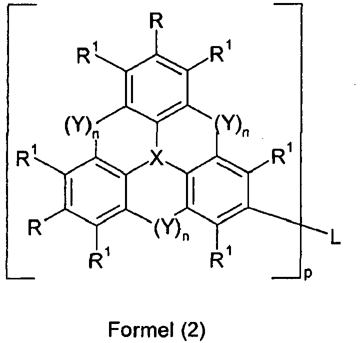 wobei die verwendeten Symbole und Indizes dieselbe Bedeutung haben, wie in Anspruch 1 beschrieben.

## Claims

1. Organic electroluminescent device comprising anode, cathode and at least one emitting layer, **characterised in that** at least one layer comprises at least one compound of the formula (1) or formula (2), 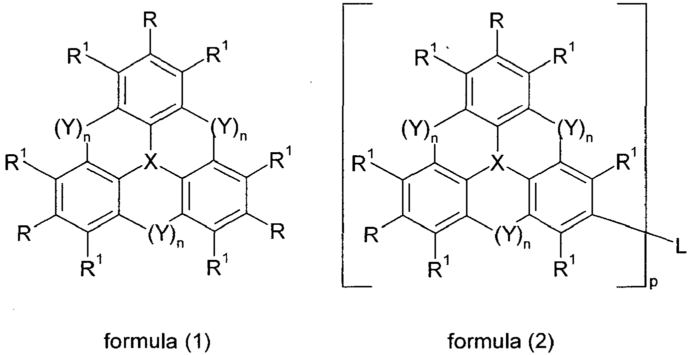 where the following applies to the symbols and indices used:
X is on each occurrence, identically or differently, N, P, As, Sb, P=O, As=O or Sb=O;
Y is, identically or differently on each occurrence, O, S, C(R¹)₂, C=C(R¹)₂, Si(R¹)₂, BR¹, NR¹, PR¹, AsR¹, SbR¹, BiR¹, P(=O)R¹, As(=O)R¹, Sb(=O)R¹, Bi(=O)R¹, SO, SeO, TeO, SO₂, SeO₂, TeO₂ or a chemical bond;
R¹ is on each occurrence, identically or differently, H, OH, F, Cl, Br, I, CN, NO₂, N(Ar)₂, Si(R²)₃, B(OR²)₂, C(=O)Ar, P(=O)Ar₂, S(=O)Ar, S(=O)Ar₂, CR²=CR²Ar, C≡CAr, OSO₂R², a straight-chain alkyl group having 1 to 40 C atoms or a branched or cyclic alkyl group having 3 to 40 C atoms, each of which may be substituted by one or more radicals R², where one or more non-adjacent CH₂ groups may be replaced by -R²C=CR²-, -C≡C-, Si(R²)₂, Ge(R²)₂, Sn(R²)₂, C=O, C=S, C=Se, C=NR², P(=O)R², S=O, SO₂, NR², -O-, -S- or -CONR²- and where one or more H atoms may be replaced by F, Cl, Br, I, CN or NO₂, or an aromatic or heteroaromatic ring system having 5 to 40 aromatic ring atoms, which may be substituted by one or more radicals R², or a combination of two, three, four or five of these systems; two or more substituents R¹ here, both on the same ring and also on different rings, may also form a mono- or polycyclic, aliphatic, aromatic or heteroaromatic ring system with one another;
R is defined like R¹, where at least one radical R is not equal to hydrogen;
R² is on each occurrence, identically or differently, H or an aliphatic, aromatic and/or heteroaromatic hydrocarbon radical having 1 to 20 C atoms, where two or more radicals R² may also form a ring system with one another;
Ar is on each occurrence, identically or differently, an aromatic or heteroaromatic ring system having 5-30 aromatic ring atoms, which may be substituted by one or more non-aromatic radicals R¹;
L is an at least divalent straight-chain alkylene, alkyleneoxy or thioalkyleneoxy group having 1 to 40 C atoms or a branched or cyclic alkylene, alkyleneoxy or thioalkyleneoxy group having 3 to 40 C atoms, each of which may be substituted by one or more radicals R², where one or more non-adjacent CH₂ groups may be replaced by -CR²=CR²-, -C≡C-, Si(R²)₂, Ge(R²)2, Sn(R²)2, C=O, C=S, C=Se, C=NR², P(=O)R², S=O, SO₂, -O-, -S- or -CONR²- and where one or more H atoms may be replaced by F, Cl, Br, I, CN or NO₂, or an at least divalent aromatic or heteroaromatic ring system having 5 to 40 aromatic ring atoms, which may be substituted by one or more radicals R², or P(R¹)₃₋ₚ, P(=O)(R¹)₃₋ₚ, C(R¹)₄₋ₚ, Si(R¹)₄₋ₚ, N(Ar)₃₋ₚ or a combination of two, three, four or five of these systems; or L is a chemical bond;
n is on each occurrence, identically or differently, 0, 1 or 2, where n = 0 means that a hydrogen or radical R¹ is present instead of Y, with the proviso that at least two indices n are not equal to 0;
p is 2, 3, 4, 5 or 6, with the proviso that p is not greater than the maximum valency of L;
the following compound is excluded from the invention:

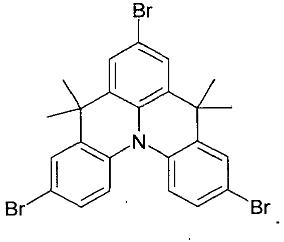

2. Organic electroluminescent device according to Claim 1, **characterised in that** the symbol X stands for nitrogen, phosphorus or P=O.

3. Organic electroluminescent device according to Claim 1 or 2, **characterised in that** the symbols Y stand, identically or differently on each occurrence, for O, S, (CR¹)₂, P(=O)R¹; C=C(R¹)₂, NR¹, SO, SO₂ or a chemical bond.

4. Organic electroluminescent device according to one or more of Claims 1 to 3, **characterised in that** the symbol R¹ stands, identically or differently on each occurrence, for H, F, CF₃, an aliphatic, aromatic or heteroaromatic hydrocarbon radical having 1 to 10 C atoms, or **in that** R¹ on bridges Y stands for an aliphatic hydrocarbon radical having 1 to 6 C atoms or for an aryl or heteroaryl group having 6 to 10 C atoms, and two radicals R¹ on the same bridge Y may form a ring system with one another.

5. Organic electroluminescent device according to one or more of Claims 1 to 4, **characterised in that** the symbol R stands on each occurrence, identically or differently, for F, Cl, Br, I, CHO, B(OR²)₂, P(R²)₂, N(Ar)₂, CR²=CR²Ar, C≡CAr or an aromatic or heteroaromatic ring system having 5 to 30 aromatic ring atoms, which may be substituted by one or more non-aromatic radicals R¹, or a combination of two, three, four or five of these radicals.

6. Organic electroluminescent device according to one or more of Claims 1 to 5, **characterised in that** the symbol L stands for -R²C=CR²-, -C≡C-, C=O, S=O, SO₂, -O-, -S-, P(R¹)₃₋ₚ, P(=O)(R¹)₃₋ₚ, C(R¹)₄₋ₚ, Si(R¹)₄₋ₚ, N(Ar)₃₋ₚ or an aromatic or heteroaromatic ring system having 6 to 30 aromatic ring atoms, which may be substituted by one or more radicals R², or a combination of two, three or four of these systems or a chemical bond.

7. Organic electroluminescent device containing at least one polymer, oligomer or dendrimer containing one or more of the compounds of the formula (1) or formula (2), where at least one radical R or R¹ represents a bond to the polymer.

8. Organic electroluminescent device according to one or more of Claims 1 to 7, **characterised in that** the compound of the formula (1) or formula (2) is employed as a mixture with at least one host material and/or **in that** the compound of the formula (1) or formula (2) is employed as hole-transport and/or hole-injection material.

9. Use of compounds of the formula (1) or formula (2) according to Claim 1 in organic electroluminescent devices.

10. Compound of the formula (2), 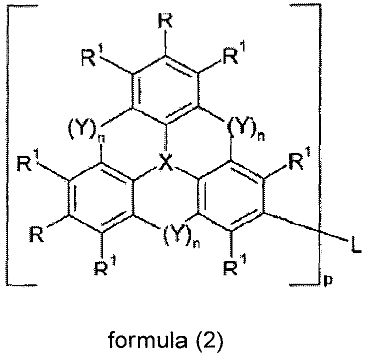 where the symbols and indices used have the same meaning as described in Claim 1.

## Revendications

1. Dispositif électroluminescent organique comprenant une anode, une cathode et au moins une couche d'émission, **caractérisé en ce qu'**au moins une couche comprend au moins un composé de la formule (1) ou de la formule (2) : 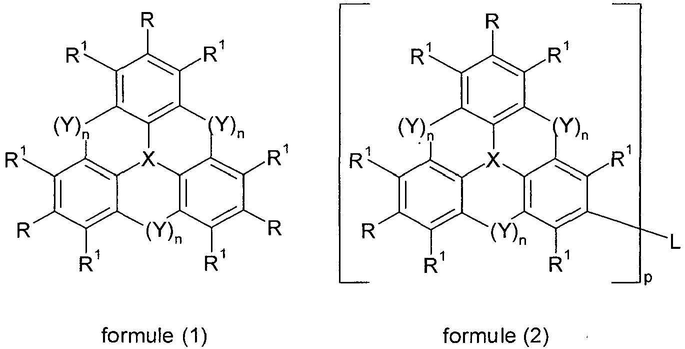 dans lesquelles ce qui suit s'applique aux symboles et indices utilisés :
X est pour chaque occurrence, de manière identique ou différente, N, P, As, Sb, P=O, As=O ou Sb=O ;
Y est, de manière identique ou différente pour chaque occurrence, O, S, C(R¹)₂, C=C(R¹)₂, Si(R¹)₂, BR¹, NR¹, PR¹, AsR¹, SbR¹, BiR¹, P(=O)R¹, As(=O)R¹, Sb(=O)R¹, Bi(=O)R¹, SO, SeO, TeO, SO₂, SeO₂, TeO₂ ou une liaison chimique ;
R¹ est pour chaque occurrence, de manière identique ou différente, H, OH, F, Cl, Br, I, CN, NO₂, N(Ar)₂, Si(R²)₃, B(OR²)₂, C(=O)Ar, P(=O)Ar₂, S(=O)Ar, S(=O)Ar₂, CR²=CR²Ar, C≡CAr, OSO₂R², un groupe alkyle en chaîne droite qui comporte 1 à 40 atome(s) de C ou un groupe alkyle ramifié ou cyclique qui comporte 3 à 40 atomes de C, dont chacun peut être substitué par un radical ou plusieurs radicaux R², où un ou plusieurs groupe(s) CH₂ non adjacents peut/peuvent être remplacé(s) par -R²C=CR²-, -C≡C-, Si(R²)₂, Ge(R²)₂, Sn(R²)₂, C=O, C=S, C=Se, C=NR², P(=O)R², S=O, SO₂, NR², -O-, -S- ou -CONR²- et où un ou plusieurs atome(s) de H peut/peuvent être remplacé(s) par F, Cl, Br, I, CN ou NO₂, ou un système de cycle aromatique ou hétéroaromatique comportant 5 à 40 atomes de cycle aromatique, lequel peut être substitué par un radical ou plusieurs radicaux R², ou une combinaison de deux, trois, quatre ou cinq de ces systèmes ; deux substituants R¹ ou plus ici, à la fois sur le même cycle et également sur des cycles différents, peuvent également former un système de cycle aliphatique, aromatique ou hétéroaromatique monocyclique ou polycyclique l'un avec l'autre ou les uns avec les autres ;
R est défini de façon similaire à R¹, où au moins un radical R n'est pas égal à hydrogène ;
R² est pour chaque occurrence, de manière identique ou différente, H ou un radical hydrocarbone aliphatique, aromatique et/ou hétéroaromatique qui comporte 1 à 20 atome(s) de C, où deux radicaux R² ou plus peuvent également former un système de cycle l'un avec l'autre ou les uns avec les autres ;
Ar est pour chaque occurrence, de manière identique ou différente, un système de cycle aromatique ou hétéroaromatique qui comporte 5 à 30 atomes de cycle aromatique, lequel peut être substitué par un radical ou plusieurs radicaux non aromatique(s) R¹ ;
L est au moins un groupe alkylène, alkylèneoxy ou thioalkylèneoxy en chaîne droite divalent qui comporte 1 à 40 atome(s) de C ou un groupe alkylène, alkylèneoxy ou thioalkylèneoxy ramifié ou cyclique qui comporte 3 à 40 atomes de C, dont chacun peut être substitué par un radical ou plusieurs radicaux R², où un ou plusieurs groupe(s) CH₂ non adjacents peut/peuvent être remplacé(s) par -CR²=CR²-, -C≡C-, Si(R²)₂, Ge(R²)2, Sn(R²)2, C=O, C=S, C=Se, C=NR², P(=O)R², S=O, SO₂, -O-, -S- ou -CONR²-et où un ou plusieurs atome(s) de H peut/peuvent être remplacé(s) par F, Cl, Br, I, CN ou NO₂, ou au moins un système de cycle aromatique ou hétéroaromatique divalent qui comporte 5 à 40 atomes de cycle aromatique, lequel peut être substitué par un radical ou plusieurs radicaux R², ou P(R¹)₃₋ₚ, P(=O)(R¹)₃₋ₚ, C(R¹)₄₋ₚ, Si(R¹)₄₋ₚ, N(Ar)₃₋ₚ ou une combinaison de deux, trois, quatre ou cinq de ces systèmes ; ou L est une liaison chimique ;
n est pour chaque occurrence, de manière identique ou différente, 0, 1 ou 2, où n = 0 signifie qu'un hydrogène ou un radical R¹ est présent en lieu et place de Y, étant entendu qu'au moins deux indices n ne sont pas égaux à 0 ;
p est 2, 3, 4, 5 ou 6, étant entendu que p n'est pas supérieur à la valence maximum de L ;
le composé qui suit est exclus de l'invention :

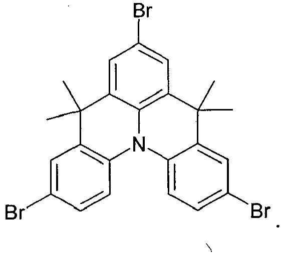

2. Dispositif électroluminescent organique selon la revendication 1, **caractérisé en ce que** le symbole X représente azote, phosphore ou P=O.

3. Dispositif électroluminescent organique selon la revendication 1 ou 2, **caractérisé en ce que** les symboles Y représentent, de manière identique ou différente pour chaque occurrence, O, S, (CR¹)₂, P(=O)R¹, C=C(R¹)₂, NR¹, SO, SO₂ ou une liaison chimique.

4. Dispositif électroluminescent organique selon une ou plusieurs des revendications 1 à 3, **caractérisé en ce que** le symbole R¹ représente, de manière identique ou différente pour chaque occurrence, H, F, CF₃, un radical hydrocarbone aliphatique, aromatique ou hétéroaromatique qui comporte 1 à 10 atome(s) de C, ou **en ce que** R¹ sur des ponts Y représente un radical hydrocarbone aliphatique qui comporte 1 à 6 atome(s) de C ou un groupe aryle ou hétéroaryle qui comporte 6 à 10 atomes de C, et deux radicaux R¹ sur le même pont Y peuvent former un système de cycle l'un avec l'autre.

5. Dispositif électroluminescent organique selon une ou plusieurs des revendications 1 à 4, **caractérisé en ce que** le symbole R représente pour chaque occurrence, de manière identique ou différente, F, CI, Br, I, CHO, B(OR²)₂, P(R²)₂, N(Ar)₂, CR²=CR²Ar, C≡CAr ou un système de cycle aromatique ou hétéroaromatique qui comporte 5 à 30 atomes de cycle aromatique, lequel peut être substitué par un radical ou plusieurs radicaux non aromatique(s) R¹, ou une combinaison de deux, trois, quatre ou cinq de ces radicaux.

6. Dispositif électroluminescent organique selon une ou plusieurs des revendications 1 à 5, **caractérisé en ce que** le symbole L représente -R²C=CR²-, -C≡C-, C=O, S=O, SO₂, -O-, -S-, P(R¹)₃₋ₚ, P(=O)(R¹)₃₋ₚ, C(R¹)₄₋ₚ, Si(R¹)₄₋ₚ, N(Ar)₃₋ₚ ou un système de cycle aromatique ou hétéroaromatique qui comporte 6 à 30 atomes de cycle aromatique, lequel peut être substitué par un radical ou plusieurs radicaux R², ou une combinaison de deux, trois ou quatre de ces systèmes ou une liaison chimique.

7. Dispositif électroluminescent organique contenant au moins un polymère, un oligomère ou un dendrimère contenant un ou plusieurs des composés de la formule (1) ou de la formule (2), où au moins un radical R ou R¹ représente une liaison sur le polymère.

8. Dispositif électroluminescent organique selon une ou plusieurs des revendications 1 à 7, **caractérisé en ce que** le composé de la formule (1) ou de la formule (2) est utilisé en tant que mélange avec au moins un matériau hôte et/ou **en ce que** le composé de la formule (1) ou de la formule (2) est utilisé en tant que matériau de transport de trous et/ou d'injection de trous.

9. Utilisation de composés de la formule (1) ou de la formule (2) selon la revendication 1 dans des dispositifs électroluminescents organiques.

10. Composé de la formule (2) : 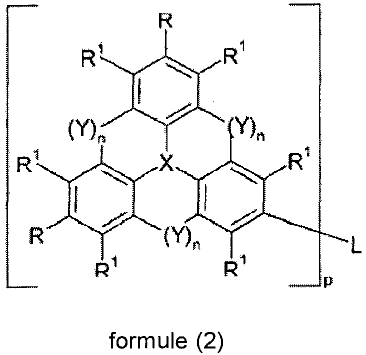 dans laquelle les symboles et indices utilisés présentent la même signification que décrit selon la revendication 1.
